# EUROPEAN PATENT SPECIFICATION

(11) **EP 3 861 139 B1**
(45) Date of publication and mention of the grant of the patent: **07.08.2024**
(21) Application number: 19790797.5
(22) Date of filing: 24.09.2019
(51) Int. Cl.: C12Q 1/6886

(54) **IDENTIFICATION OF MOLECULAR BIOMARKERS THAT ARE PREDICTIVE OF THE RESPONSE TO RADIOCHEMOTHERAPY TREATMENT IN CERVIX CARCINOMA**
IDENTIFIZIERUNG VON MOLEKULAREN BIOMARKERN, DIE PRÄDIKTIV FÜR DAS ANSPRECHEN AUF EINE RADIOCHEMOTHERAPIE BEI ZERVIXKARZINOM SIND
IDENTIFICATION DE BIOMARQUEURS MOLÉCULAIRES PRÉDICTIFS DE LA RÉPONSE À UN TRAITEMENT DE RADIOCHIMIOTHÉRAPIE DANS LE CARCINOME DU COL DE L'UTÉRUS

(30) Priority: 03.10.2018 IT 201800009133
(43) Date of publication of application: 11.08.2021
(73) Proprietor: Università Cattolica del Sacro Cuore, 20123 Milano MI (IT); Agenzia Nazionale Per Le Nuove Tecnologie, L'Energia E Lo Sviluppo Economico Sostenibile (ENEA), 00196 Roma (IT)
(72) Inventor: SCAMBIA, Giovanni, 20123 Milano (IT); GALLO, Daniela, 20123 Milano (IT); FERRANDINA, Maria Gabriella, 20123 Milano (IT); PETRILLO, Marco, 20123 Milano (IT); RASPAGLIO, Giuseppina, 20123 Milano (IT); BUTTARELLI, Marianna, 20123 Milano (IT); MANCUSO, Mariateresa, 00196 Roma (IT); SARAN, Anna, 00196 Roma (IT); MARINO, Carmela, 00196 Roma (IT); DESIDERIO, Angiola, 00196 Roma (IT); VILLANI, Maria Elena, 00196 Roma (IT)
(74) Representative: Di Giovine, Paolo
(86) International application number: PCT/IB2019/058075
(87) International publication number: WO 2020/070582

(56) References cited:
- EP-A2- 2 195 005
- DATABASE MEDLINE [online] US NATIONAL LIBRARY OF MEDICINE (NLM), BETHESDA, MD, US; March 2018 (2018-03-01), JOSAHKIAN JULIANA A ET AL: "Increased STAT1 Expression in High Grade Serous Ovarian Cancer Is Associated With a Better Outcome.", Database accession no. NLM29303938
- JOSAHKIAN JULIANA A ET AL: "Increased STAT1 Expression in High Grade Serous Ovarian Cancer Is Associated With a Better Outcome.", INTERNATIONAL JOURNAL OF GYNECOLOGICAL CANCER : OFFICIAL JOURNAL OF THE INTERNATIONAL GYNECOLOGICAL CANCER SOCIETY 03 2018, vol. 28, no. 3, March 2018 (2018-03-01), pages 459 - 465, ISSN: 1525-1438
- KITAHARA OSAMU ET AL: "Classification of sensitivity or resistance of cervical cancers to ionizing radiation according to expression profiles of 62 genes selected by cDNA microarray analysis", NEOPLASIA, DOYMA, BARCELONA, ES, vol. 4, no. 4, 1 July 2002 (2002-07-01), pages 295 - 303, XP002405529, ISSN: 0212-9787
- NISHIO S ET AL: "Cap43/NDRG1/Drg-1 is a molecular target for angiogenesis and a prognostic indicator in cervical adenocarcinoma", CANCER LETTERS, NEW YORK, NY, US, vol. 264, no. 1, 8 June 2008 (2008-06-08), pages 36 - 43, XP022625726, ISSN: 0304-3835, [retrieved on 20080220], DOI: 10.1016/J.CANLET.2008.01.020
- DATABASE MEDLINE [online] US NATIONAL LIBRARY OF MEDICINE (NLM), BETHESDA, MD, US; March 2018 (2018-03-01), JOSAHKIAN JULIANA A ET AL: "Increased STAT1 Expression in High Grade Serous Ovarian Cancer Is Associated With a Better Outcome.", XP002792177, Database accession no. NLM29303938
- INTERNATIONAL JOURNAL OF GYNECOLOGICAL CANCER : OFFICIAL JOURNAL OF THE INTERNATIONAL GYNECOLOGICAL CANCER SOCIETY 03 2018, vol. 28, no. 3, March 2018 (2018-03-01), pages 459 - 465, ISSN: 1525-1438
- LANYING JIN ET AL: "Immunohistochemical expression of Annexin A2 and S100A proteins in patients with bulky stage IB IIA cervical cancer treated with neoadjuvant chemotherapy", GYNECOLOGIC ONCOLOGY, ACADEMIC PRESS, LONDON, GB, vol. 126, no. 1, 2 April 2012 (2012-04-02), pages 140 - 146, XP028494216, ISSN: 0090-8258, [retrieved on 20120406], DOI: 10.1016/J.YGYNO.2012.04.005
- MARIANNA BUTTARELLI ET AL: "A combined ANXA2-NDRG1-STAT1 gene signature predicts response to chemoradiotherapy in cervical cancer", JOURNAL OF EXPERIMENTAL & CLINICAL CANCER RESEARCH, vol. 38, no. 1, 26 June 2019 (2019-06-26), XP055653432, DOI: 10.1186/s13046-019-1268-y

## Description

The present invention relates to the identification of new markers and methods predictive of the response to radiochemotherapy treatment in cervix carcinoma, their use and the use of kits comprising them to predict the response to radiochemotherapy treatment in cervix carcinoma.

### STATE OF THE PRIOR ART

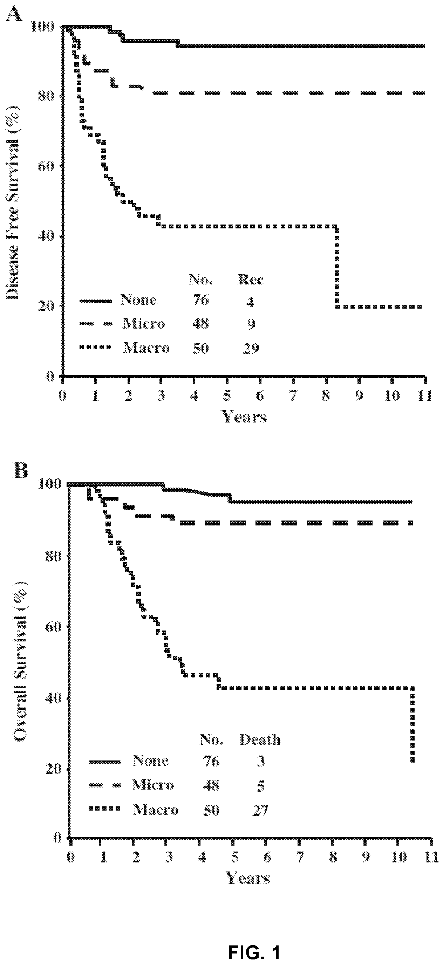

Cervix carcinoma is the fourth most common tumor in women, with an estimate of 530,000 new cases per year. Moreover, every year over 270.000 deaths due to cancer of the neck of the uterus are recorded, over 85% of which in less-developed regions [GLOBOCAN 2012 (IARC)] Section of Cancer Information (23/01/2017)]. Most patients with early stage disease are subjected to exclusive treatment with surgery or radiation therapy, whereas for patients with IB2 to IVA stages of the disease radiochemotherapy is the standard treatment (Monk et al., 2007; Gaffney and Soisson, 2010). Cisplatin is the drug commonly used in association with radiotherapy, both alone, and in combination with other agents such as 5-fluorouracil (5-FU) or hydroxyurea. Patients with advanced disease are at a greater risk of recurrence and represent the greater share of deaths due to cervix carcinoma, with an overall 5-year survival of about 70% (Eifel et al., 2004, Cochrane database 2010; Noordhuis et al., 2011). Therefore, although the concomitant radiochemotherapy treatment has significantly improved disease control and survival, for patients with an advanced disease other approaches were evaluated as well, among which post-treatment completion surgery [Classe et al., 2006; Ferrandina et al., 2007, Motton et al., 2010]. In fact, the introduction of surgery after a radiochemotherapy treatment represents a potential advantage, as it enables to remove radiochemoresistant tumor residues with local control and a better survival. Moreover, this approach provides information on the pathological response to the treatment (evaluation by histological examination of the residual tumor, after examination of the entire cervix) defined as complete (in the absence of tumor residue), microscopic (in case of residual foci with a <3mm extension) and macroscopic (in case of foci with a >3mm extension) (Ferrandina et al., 2007). It is important to stress that a long-term analysis on patients in treatment at our Department demonstrated how the radiochemotherapy treatment followed by radical surgery can yield encouraging results in terms of disease-free time and overall survival in the entire series of LACC (Locally Advanced Cervical Cancers) patients and in stage III-IVA disease subgroup (Ferrandina et al., 2010). However, tumor cell resistance to radiochemotherapy remains the main therapeutic drawback, and about 30% of patients shows a partial response or stable disease after the radiochemotherapy protocol, with an unfavorable overall survival. As is deduced from Figure 1, the possibility to obtain a complete or micro- response to radiochemotherapy treatment confers a considerable clinical advantage to LACC patients. These data highlight the critical role of sensitivity or resistance to radiochemotherapy in LACC patients, with literature evidences supporting a genetic basis for cell radiochemoresistance, even though the associated biochemical and molecular events have not been fully understood. Clinical-pathological factors, including tumor stage and histology, as well as the most sophisticated imaging techniques, such as MRI and PET-CT, can serve as response markers to radiochemotherapy treatment, but are not fully predictive of the variabilities observed. Various mechanisms have been implicated in the cells' adaptive radiotherapy response, and studies on molecular biomarkers validated in clinical studies have led to the identification of gene products relevant to specific biological functions such as apoptosis, cell adhesion, DNA repair, hypoxia, metabolism, pluripotency and proliferation (Kilic et al., 2015). As many studies were carried out on *in vitro* systems, such as stabilized tumor cell lines or primary cultures from radiotreated patients (Kilic et al., 2015).

Moreover, in order to identify biological markers predictive of response to radiochemotherapy, the genetic expression profile of patients with advanced-stage cervix carcinoma was characterized (Kitahara et al., 2002; Harima et al., 2009). Even though no definitive result has been achieved as yet, there are data supporting a role of the signaling pathways linked to EGFR, C-erbB-2, and COX-2 in radiochemotherapy resistance mechanisms (Noordhuis et al., 2011). In fact, owing to tumor heterogeneity, it is unlikely that a specific marker may have a prognostic or predictive value, whereas an expression profile could enable the identification of a phenotype of cell radiochemoresistance or sensitivity. EP 2 195 005 discloses an in vitro method of predicting the sensitivity of a cell to a selected dose of radiation therapy using a subset of signature genes comprising STAT1.

Therefore, the identification of a molecular profile of resistance or sensitivity to radiochemotherapy treatment is of remarkable clinical interest as a tool for the identification of customized therapeutic strategies in the treatment of cervix carcinoma, potentially more effective and less toxic. Therefore, new methods of prognosis of cervix carcinoma, whose results may provide accurate indications on responsiveness to radiochemoresistance, are desirable.

### SUMMARY OF THE INVENTION

The present invention is based on the identification of a panel of biomarkers predictive of resistance or sensitivity of cervix carcinoma cells to radiochemotherapy treatment. As described in detail in the experimental section of the present description, in order to identify such biomarkers biopsy samples were collected from LACC patients, who were subsequently subjected to neoadjuvant radiochemotherapy treatment, followed by surgery. Based on the pathological response identified during surgery as complete (absence of tumor residue), and macroscopic (neoplastic foci with a >3mm extension), patients were subdivided into two groups (respectively, **S**=sensitive; **R**=resistant; n=20 patients/group). The selected biopsies were processed for extraction of proteins and nucleic acids to be used for subsequent analyses. From a differential proteomic analysis that compared protein expression profiles of biopsies from therapy-resistant patients with those from therapy-sensitive patients, there were identified 25 proteins of potential interest for their involvement in cervix carcinoma and/or in the mechanisms of response to radiochemotherapy treatments. The expression variations detected were also confirmed by nanofluidic-based qPCR analysis of transcripts corresponding to the 25 proteins selected by proteomic analysis. The obtained results were subjected to bioinformatics computing (data processing), which allowed to identify in a statistically rigorous way 3 genes, ANXA2, NDRG1 and STAT1, differently expressed between the two groups S and R. Therefore, the Authors of the present invention have identified a panel of three genes differently expressed in LACC **S** and **R** patients, allowing to predict the probability of response to radiochemotherapy treatment individually for each patient. An adequate stratification of the patients is consistent with a customized medicine approach. In other words, if the algorithm's prediction will assign to that particular patient a sensitivity phenotype, the patient will follow a standard therapeutic approach; patients with a resistant phenotype will instead be subjected to therapies alternative to radiochemotherapy treatment, avoiding not only ineffective, but potentially damaging treatments. Therefore, the method proposed in the present invention not only improves the treatment of the pathology but enables to reduce the direct costs of treatment (e.g., for a lower hospitalization), as well as the indirect ones associated with the disease (social costs, productivity loss, etc.).

The invention therefore relates to:
- a method, *in vitro* or *ex vivo,* for the prognosis in a patient with cervix carcinoma comprising:
   a) determining, in a sample obtained from said patient, the level of expression of the following markers: ANXA2, STAT1 and NDRG1;
   b) comparing the level of expression determined in step a) with one or more reference values.
- a method, *in vitro* or *ex vivo,* to predict the responsiveness or non-responsiveness to a radiochemotherapy, radiotherapy and/or chemotherapy treatment in a patient with cervix carcinoma, comprising:
   a) determining, in a sample obtained from said patient, the level of expression of the following markers: ANXA2, STAT1 and NDRG1;
   b) comparing the level of expression determined in step a) with one or more reference values.
- a kit for the prognosis of cervix carcinoma, in particular to predict the responsiveness or non-responsiveness to a radiochemotherapy treatment, comprising agents for measuring the expression level of mRNA or proteins of the following markers: ANXA2, STAT1 and NDRG1.
- the use of the following markers: ANXA2, STAT1 and NDRG1 for the prognosis of cervix carcinoma, in particular to predict the responsiveness or non-responsiveness to a radiochemotherapy treatment in a patient with cervix carcinoma.

### BRIEF DESCRIPTION OF THE FIGURES

**Figure 1****.** Clinical outcome in LACC patients, related to pathological response (i.e., none = absence of disease; micro = ≤ 3 mm residue; macro = > 3 mm residue) (Ferrandina et al., 2010).
**Figure 2****.** Differential protein spots identified following 2D-DIGE analysis.
**Figure 3****.** Proteomic analysis results were subjected to multivaried analysis (PCA). PCA is an analysis enabling to visualize the degree of variability between the two groups of samples being compared. The result of said analysis highlighted a significant on-plot separation between the samples belonging to the group of sensitive patients and those belonging to the group of resistant patients, an index of the presence of protein differences between the two groups.
**Figure 4****.** Box diagram describing, for each of the 25 gene transcripts examined, ΔCt (Ct of the gene of interest - Ct of the reference gene) distribution in the two groups of patients analyzed, S and R (n = 16 for both groups).
**Figure 5****.** Volcano plot analysis allows to visualize differently expressed genes with lowest p values. As is deduced from the graph, NDRG1, ANXA2, and STAT1 are the 3 genes with statistically significant differences in fold change values between the two groups of patients analyzed, S and R (n=16 for both groups).
**Figure 6****.** Box diagram describing for ANXA2, NDRG1 and STAT1 the number of copies of the gene (as copies/µl of cDNA) obtained by use of DigitalPCR in the two groups of patients analyzed, S and R (n = 10 for both groups).
**Figure 7****.** Volcano plot analysis related to ANXA2, NDRG1 and STAT1 analyzed by RT-qPCR. As is deduced from the graph, the 3 genes identified in nanofluidic-based qPCR maintain statistically significant differences in fold change values between the two groups of patients analyzed, S and R (n=16 and 13, respectively).
**Figure 8****:** Ingenuity Pathway Analysis (IPA) for the identification of groups of associated genes. As is deduced from the figure, ANXA2, NDRG1 and STAT1 converge on the p53 pathway.

### DETAILED DESCRIPTION OF THE INVENTION

As already described above, the present invention provides a new marker to predict the prognosis of cervix carcinoma, wherein the marker is a combination of ANXA2, STAT1 and NDRG1, in particular to predict the responsiveness or non-responsiveness to an antitumor treatment such as radiochemotherapy.

In one aspect, the present invention refers to a method for the prognosis in a patient with cervix carcinoma, in particular to predict the responsiveness or non-responsiveness to an antitumor treatment such as radiochemotherapy, radiotherapy and/or chemotherapy, comprising the following steps:
a) determining, in a sample obtained from said patient, the level of expression of the following markers: ANXA2, STAT1 and NDRG1;
b) comparing the level of expression determined in step a) with a reference value. By the term "ANXA2" we are referring to Annexin A2, whose human gene, the corresponding mRNA and encoded protein are available, e.g., in the Ensembl data bank with code ENSG00000182718.

By the term "STAT1" we are referring to a signal transducer and transcription activator 1, whose human gene, the corresponding mRNA and encoded protein are available, e.g., in the Ensembl data bank with code ENSG00000115415.

By the term "NDRG1" we are referring to the member of the family of downregulated N-myc genes whose human gene, the corresponding mRNA and encoded protein are available, e.g., in the Ensembl data bank with code ENSG00000104419.

As used herein, the term "marker" or "biomarker" refers to a molecule quantitatively or qualitatively associated with the presence of biological phenomena, and the marker of the present invention refers to the gene which is the basis to predict cervix carcinoma patients with a positive or negative prognosis, in particular to predict the responsiveness or non-responsiveness to a radiochemotherapy treatment.

As used herein, the term "prognosis" refers to the expectancy on the medical development (e.g., the possibility of long-term survival, disease-free survival rate, etc.), includes positive prognosis or negative prognosis, negative prognosis includes disease progression as recurrence, tumor growth, metastasis and mortality from resistance to drugs, and positive prognosis includes disease remission or stabilization following a suitable therapy.

As used herein, the term "predict" refers to the assumption on the medical development and, for the object of the present invention, probability of disease development (disease progression, improvement, cervix carcinoma recurrence, tumor growth) in patients with a diagnosis of cervix carcinoma.

The sample used in the method according to the present invention is obtained from a patient suffering from carcinoma and may be, but not limited to, a biopsy sample of tissue (cells), originated from the uterine cervix of said patient, preferably from tumor tissue. The patient from which the sample to be analyzed is obtained will be, e.g., a patient with locally advanced cervix carcinoma (i.e., LACC of stage from IB2 to IVA).

In one aspect of the present invention the method will be used to predict the responsiveness or non-responsiveness to an antitumor treatment such as radiochemotherapy, radiotherapy and/or chemotherapy. In particular, in the present experimentation the samples analyzed were collected from patients subjected to neoadjuvant radiochemotherapy treatment. Patients received a local radiant treatment (at the pelvis) with a dose ranging from 39.6 to 50.4 Gy, in association with chemotherapeutic drugs such as, e.g., cisplatin, 5-fluorouracil or capecitabine, in accordance with specific protocols (e.g., Ferrandina et al., Gynecol Oncol 2007; Ferrandina et al., Gynecol Oncol 2010). At +7-8 weeks from the end of the radiochemotherapy treatment, all patients were subjected to radical hysterectomy and pelvic ± aortic lymphadenectomy.

In the method of the present invention the level of expression of the ANXA2, STAT1 and NDRG1 markers is preferably determined by measuring the mRNA and/or the corresponding protein of said markers in the sample to be analyzed. The mRNA expression level of said markers is measured by using agents like primers or probes that specifically bind to the sequences of said markers and/or wherein the level of protein expression is measured by using an antibody specific to the corresponding protein of said markers. As used herein, the term "agent for measuring the levels of expression of the markers" refers to a molecule that can be used to determine the levels of expression of the marker genes or proteins encoded by these genes, and can be preferably the antibody, primer or probe that is specific for the markers. As used herein, the term "measuring the expression level of mRNA" refers to the process to identify the presence of mRNA of the marker gene in the biologic sample and the level of expression thereof in order to predict the prognosis of cervix carcinoma by measuring the mRNA. Analysis methods are, e.g., but not limited to, RT-PCR, real time RT-PCR, Northern blotting, DNA chip/microarray, etc.

According to one embodiment, the level of expression of the markers of interest ANXA2, STAT1 and NDRG1 is normalized by comparing with the level of expression of one or more reference genes selected from the group consisting of the reference genes ACTB, B2M, GAPDH, HPRT1, PGK1 and/or RPLP0, preferably with respect to B2M.

Once determined/measured the level of expression of the ANXA2, STAT1 and NDRG1 markers in the sample, the method of the present invention envisages a second step b) in which the levels of expression measured in step a) are compared with one or more reference values to predict the prognosis of the patient whose sample was analyzed, in particular to predict what her responsivity to the antitumor treatment, in particular to radiochemotherapy treatment, will be. As reference values there could be, e.g., considered the means of the ΔCt values (ΔCt = Ct gene of interest - Ct reference gene) of the three genes under examination, obtained from the group of (therapy-)sensitive patients analyzed in the present research. The means of the ΔCt values for ANXA2, NDRG1 and STAT1 obtained for sensitive patients, under the experimental conditions of the Inventors, were equal to 2.36 ± 0.42, 4.76 ± 0.52, 5.12 ± 0.22 (mean ± SEM), whereas said values were determined equal to 1.11 ± 0.43, 2.26 ± 0.55 and 6.25 ± 0.25, for (therapy-)resistant patients. It has to be considered that the lower the ΔCt value, the greater the absolute level of gene expression. Therefore, the reported data indicate that, compared to a sensitive patient, the pre-treatment biopsy sample from a resistant patient will express higher levels of ANXA2 and NDRG1 and lower levels of STAT1. However, the above-reported means of ΔCt values may not constitute an absolute reference value, as subjected to variations depending on the reference gene/s and on the number of analyzed samples from which these means derive. Moreover, the level of relative expression of the three genes is important, and therefore the algorithm was trained to the prediction of the phenotype of sensitivity/resistance to radiochemotherapy treatment, concomitantly introducing the data related to the three genes in the population studied. In order to improve test sensitivity and specificity, the algorithm will be further trained, increasing the patients' case histories.

According to one embodiment, the reference values are the levels of expression or the expression pattern of mRNA or protein of the corresponding markers ANXA2, STAT1 and NDRG1 in one or more patients with cervix carcinoma with known prognosis, in particular whose responsiveness or non-responsiveness to antitumor treatment, in particular to radiochemotherapy treatment, is known.

According to one embodiment, the reference values can be determined by analyzing the levels of expression or the expression models of the ANXA2, STAT1 and NDRG1 markers from multiple patients with cervix carcinoma, constructing a database of the values measured from the patients with known prognosis, i.e. whose responsiveness is known, and inserting the level of expression or the expression model of the unknown patient.

According to one embodiment the prediction model was created by applying the algorithm described by Breiman, L. in Random forests. Mach. Learn. 45, 5-32 (2001). DOI 10.1023/A:1010933404324.

Through the above analysis methods, the level of expression of cervix carcinoma markers, measured in the sample from the patient whose responsiveness to a treatment is to be known, is compared with the level of expression of the same markers measured in one or more samples whose responsiveness to a treatment is known, whereby if the sample from the patient whose responsiveness is to be known exhibits a level of expression or an expression model similar to the sample with positive responsiveness, it can be determined to have a positive prognosis, and, on the contrary, if exhibiting a level of expression or an expression model similar to the samples from the patient with negative responsiveness, a negative prognosis will be had.

In particular, as mentioned above, the experimental data indicate that, compared to a sensitive patient, the pre-treatment biopsy sample from a resistant patient will express higher levels of ANXA2 and NDRG1 and lower levels of STAT1.

According to a preferred embodiment, the method could be carried out with the following steps:
- Prearranging a biopsy sample of cervix carcinoma obtained from a patient during diagnostic surgery, preferably preserved by biopsy cryopreservation;
- extracting the RNA from the biopsy sample, e.g. with a Qiagen kit according to the instructions.
- optionally, assessing RNA quantity and quality, e.g., by Nanodrop and Bioanalyzer, respectively.
- qPCR for the 3 markers of interest, ANXA2, STAT1 and NDRG1, and calculating the ΔCt with respect to the reference gene B2M.
- applying the "Random Forest" (RF) algorithm, e.g., as implemented in Orange software, to predict the response to radiochemotherapy treatment.

In another aspect, the present invention provides a kit and the use thereof to predict the prognosis of cervix carcinoma, wherein the kit comprises agents for measuring the levels of expression of mRNA or protein of markers to predict the responsiveness to radiochemotherapy. According to one embodiment, the kit of the present invention can be a RT-PCR kit, a real-time RT-PCR kit, a chip/microarray kit or a protein chip kit. According to one embodiment, the kit of the present invention can comprise primers or probes to measure the levels of expression of the markers, e.g., one or more pairs of primers in the group consisting of SEQ ID NO:1 to SEQ ID NO:62. According to one embodiment, the kit could comprise one or more of the reagents, test tube or other suitable container, buffer solution, deoxy nucleotides (dNTP), Taq-polymerase and reverse transcriptase, DNase, RNase inhibitors and sterile water. Primers or probes of the present invention can be chemically synthesized using the methods well-known in the state of the art. The kit could also comprise instructions, e.g. the link or the credentials to access to a program in which the above-described prediction model is applied.

In a further aspect, the present invention refers to method of treatment of a patient with cervix carcinoma, in particular LACC, comprising a first step *in vitro* wherein the expression of the 3 markers of interest, ANXA2, STAT1 and NDRG1, is determined and assessed according to any one of the embodiments described herein, and a second step of administering a radiochemotherapy to said patient if the gene expression data obtained in the first step indicate that said patient will be responsive to radiochemotherapy.

### EXAMPLES AND EXPERIMENTAL RESULTS

The identification of the molecular biomarkers predictive of the response to radiochemotherapy treatment in cervix carcinoma was obtained through a research project substantially comprised of the following 5 phases:

### PHASE 1 -Clinical phase

During phase 1 of the project, biopsy samples from patients with locally advanced cervix carcinoma (LACC, stage FIGO IB2-IVA) were collected (during diagnostic surgery) and cryopreserved. Patients were then subjected to radiochemotherapy treatment and subsequent surgery (at +4 weeks from end of treatment), with assessment of the pathological response, defined as complete (in the absence of tumor residue), microscopic (in case of residual foci with a <3mm extension) and macroscopic (in case of foci with a >3mm extension). To the ends of the object of the project, only biopsies from patients with a complete response (identified as S) or a macroscopic response (identified as R) were analyzed. The selected biopsies were processed for extraction from the same sample of proteins and nucleic acids to be used for subsequent analysis.

### PHASE 2 - Proteomic analysis of biopsy samples

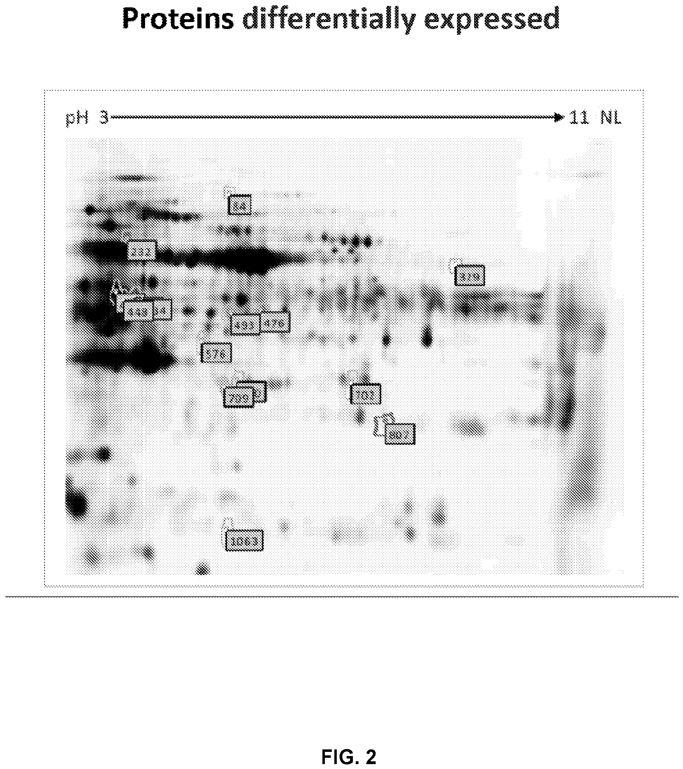

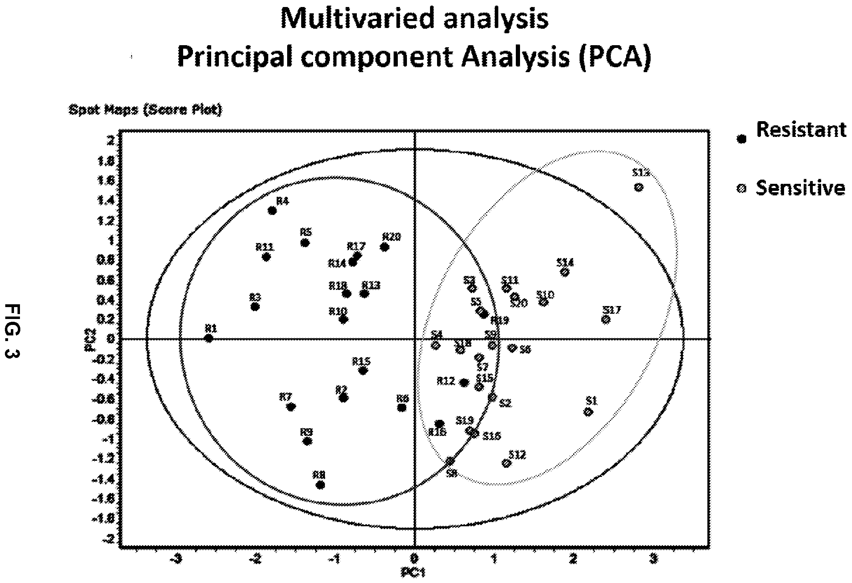

The proteins extracted from the biopsy samples were subjected to differential proteomic analysis, using 2D-DIGE technology (GE Healthcare) at the equipped ENEA facility. This analysis produced, for each biopsy sample, proteome separation profiles that were analyzed by bioinformatics computing, enabling to identify a list of proteins with variable levels of expression between the two groups S and R (Figs. 2-3). Differential protein spots were collected from gel and analyzed by mass spectrometry for identification. From the obtained list of protein annotations, 25 proteins of potential interest for their involvement in cervix carcinoma and/or in response mechanisms to radiochemotherapy treatments were selected.

### PHASE 3 - Checking of transcript modulation by nanofluidic-based qPCR.

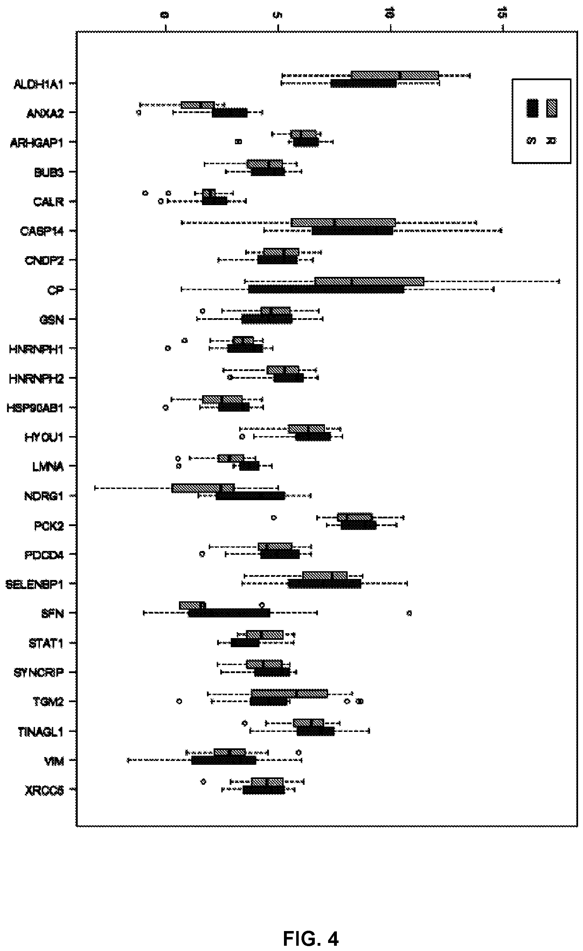

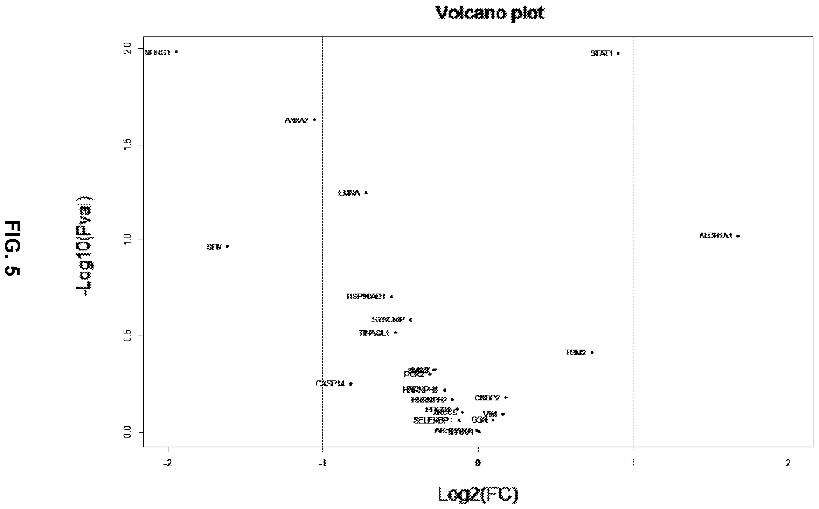

In order to check the variation of expression of the 25 proteins identified by proteomic analysis and to ascertain modulation also at transcript level, analyses were carried out by nanofluidic-based qPCR. The subsequent bioinformatics analyses allowed to confirm a differential expression between the two groups S and R for 3 genes, specifically ANXA2, NDRG1 and STAT1 (Figs. 4-5). The pattern of the abovementioned transcripts mirrors that of proteins identified from proteomic analysis in the differential protein spots listed in Table 1 (specifically, 3=STAT1; 17=NDRG1 and 19=ANXA2).

### PHASE 4 - Data confirmation with RT- qPCR analysis

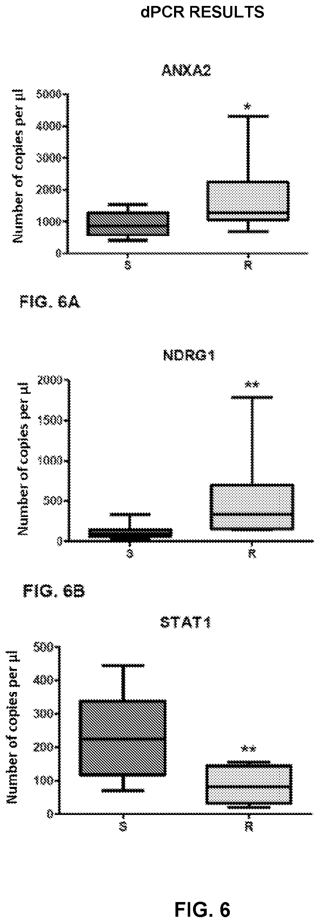

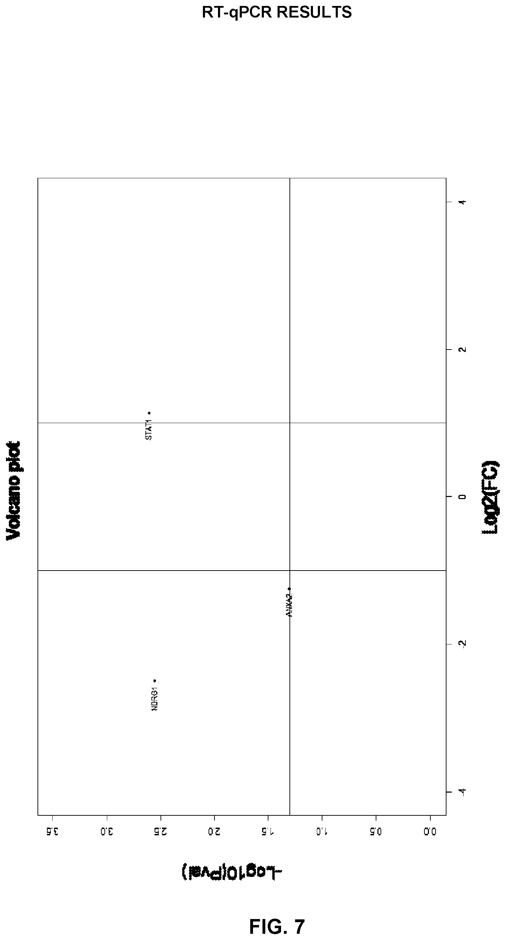

The expression of the three genes identified as significantly associated with the response to radiochemotherapy treatment was confirmed by Digital PCR technology, that allowed an absolute quantitation of the transcripts. Gene modulation was also assessed by RT-qPCR, representing the technology of reference for large-scale test development (Figs. 6-7). Finally, IPA (Ingenuity Pathway Analysis) software was used to identify the relationships among the three proteins of interest.

### PHASE 5 - Bioinformatics analysis

The dataset collected on genes ANXA2, NDRG1 and STAT1, through the RT-qPCR technique, was used as "training" dataset (dataset comprised of 29 patients: 13 Resistant "R" and 16 Sensitive "S") to implement and optimize the "Random Forest" (RF) method applied to the classification of the two groups of patients, depending on the response to the therapy. The algorithm RF "trained" on the 29 initial patients is therefore able to predict the response ("R" or "S") of new patients from the qPCR values of the genes ANXA2, NDRG1 and STAT1 obtained on biopsies collected before the therapy.

### Glossary related to some terms used in the description

Ct= threshold cycle - defined as the number of cycles required by the fluorescent signal to exceed the threshold (i.e. beyond the background level). The Ct levels are inversely proportional to the quantity of transcript in the sample.

ΔCt= difference between the Ct of the target gene and that of the reference gene. Fold change=measure of the level of expression of the transcript compared to a reference sample (in our case, compared to the mean of all samples).

### Materials and methods

### Proteomic analysis

All biopsy samples were subjected to combined extraction of DNA, RNA and proteins, by using the Qiagen "All Prep DNA/RNA/Protein Mini Kit" system. DNA, RNA and proteins were then independently purified from each individual biopsy and preserved for subsequent analysis. Protein extracts were further purified with the "2-D CleanUp Kit" (GE Healthcare) to prepare them for proteomic analysis and subsequently quantitated by "DC Protein Assay Kit" (BioRad). To assess the quality of the total purified proteins, all samples were subjected to separation by denaturing monodimensional electrophoresis. Moreover, separation tests by electrophoresis on bidimensional gel were performed to single out the optimal experimental conditions. The quantity of total proteins was normalized among the various samples, and the Inventors proceeded with fluorophore labeling (cyanins Cy5, Cy3 and Cy2) for proteomic analysis according to 2D-DIGE technology (Bidimensional Differential In Gel Electrophoresis, GE Healthcare). The various samples S and R, assessed as biological replicas of the "sensitive" and "resistant" groups, respectively, were alternately labeled with Cy5 and Cy3. Moreover, the technology envisages the introduction in the analysis of an internal standard, comprised of the pool of all samples that are labeled with a third fluorophore, Cy2. This enables to normalize the computing of the protein maps, by abating the experimental variability and reinforcing the statistical rigorousness of the results obtained. Subsequent to the labeling, proteins were separated in the first dimension by isoelectrofocalization, using the Ettan IPGphor 3 system (GE Healthcare), and isofocalization strips with a non-linear range of pH 3-11. The isofocalized samples were subsequently subjected to separation in the second dimension based on the molecular weight, by using the Ettan Dalt Twelve system (GE Healthcare). The protein profiles obtained by separation on bidimensional gel (for a total of 20 gels) were then subjected to laser scanning by using the image analyzer Typhoon 9410 (GE Healthcare). The protein maps were then loaded into the computer system for proteomic analysis using DeCyder 2D 7.2 software for the comparative computing of the samples attributed to the two groups, S and R. The 'one way-ANOVA' univariate analysis was performed by using the DeCyder-BVA (Biological Variation Analysis) module and applying the 'false discovery rate' to minimize the number of false positives. 22 protein spots with a statistically significant variability (p-value ≤ 0.05), a ≥ 1.3 difference in volume ('fold changes') and a minimum presence of 65% among protein maps (Tab.1, Fig. 2) were selected. These spots were collected from gel and analyzed by mass spectrometry, and 21 of those were identified. Multivariate analysis based on 'unsupervised nonhierarchical' (k-means procedure), 'hierarchical clustering' and 'principal component analysis' (PCA) computing was carried out using the DeCyder-EDA (Extended Data Analysis) module, in order to highlight the differences between the two groups of samples, excluding individual variability. In particular, PCA is an analysis enabling to visualize the degree of variability between the two groups of samples being compared. The result of said analysis highlighted a significant on-plot separation between samples belonging to the group of sensitive patients and those belonging to the group of resistant patients, an index of the presence of protein differences between the two groups. For mass spectrometry, proteins were reduced, S-alkylated and digested with trypsin. Thereafter, peptide fragments were subjected to analysis in a nanoLC-ESI-LIT-MS/MS LTQ XL mass spectrometer (Thermo Finnigan) equipped with a Proxeon nanospray source connected to Easy- nanoLC (Proxeon). Mascot software (Matrix Science) was used to single out correspondences between mass spectrometry raw data and human sequences in Uniprot data bank. Candidate proteins with more than two peptides assigned with a Mascot score ≥ 30 and with p-value ≤ 0.05 were considered for identification. Mass spectrometry analysis highlighted the difficulty associated with heterogeneity among the individual samples, attributable to histologic variability of biopsy collections. In fact, a multiple annotation corresponded to each single protein spot. Therefore, it was necessary to carry out an operation of selection among identification options for each protein spot. Based on data present in the literature, there were selected 25 proteins of potential interest for their involvement in cervix carcinoma and/or in response mechanisms to radiochemotherapy treatments.

### RT-nanofluidic-based qPCR

To perform gene expression analysis of the transcripts corresponding to the 25 proteins selected by proteomic analysis the RT-nanofluidic-based qPCR, Biomark HD system, Fluidigm was used. Exclusively RNA samples with a good quantity (assessed by Nanodrop spectrophotometer, Thermofisher) and quality (assessed by Bioanalyzer, Agilent) (total 32 samples, 16 per each group) were processed for subsequent analysis. The panel of DELTAgene primers (as reported in Table 2) was constructed by Fluidigm so that each pair of primers would amplify, if possible, all of the transcriptional variants corresponding to the 25 proteins singled out beforehand. From 50 ng of RNA extracted from the biopsy samples (as above) cDNA was obtained by retrotranscription, following the Fluidigm kit indications. The cDNA was pre-amplified with the panel of DELTAgene primers, with the following thermocyclic protocol: 95°C, 2 min; 14 PCR cycles of 95°C,15 sec and 60°C,4 min. Thereafter, the pre-amplified cDNA was treated with exonuclease I (New England Biolabs) (37°C, 30 min; 80°C, 15 min) and diluted 18 times. The preamplified cDNA samples were analyzed by using the 48.48 Dynamic Array IFC and the RT-nanofluidic-based qPCR, BioMark HD system. By means of the 48.48 Dynamic Array IFC it is possible to analyze gene expression by using a panel of DELTAgene primers on a maximum of 48 samples, through a nanofluidic technology applied to integrated circuits. In detail, following priming of the 48.48 IFC by the IFC Controller MX (according to Fluidigm protocol) the mix comprised of pre-amplified cDNA, 2x Sso Fast EvaGreen Supermix with Low ROX (Bio-Rad Laboratories) and 20x DNA Binding Dye (Fluidigm) was dispensed in each individual well, in the portion allotted to primers inside the 48.48 IFC. Likewise, for the panel of DELTAgene primers, each mix of primers (100 µM) and 2x assay loading reagent (Fluidigm) was dispensed in each individual well in the portion allotted to primers inside the 48.48 IFC. After the loading of the mix of primers and of samples inside the 48.48 IFC in the IFC Controller MX had taken place, the 48.48 IFC was transferred into the BioMark HD. qPCR was carried out by using the GE Fast 48x48 PCR + Melt v2.pcl protocol consisting of the following thermal steps: 95°C, 1 min; 30 PCR cycles of 96°C, 5 s; 60°C, 20 s and a melting curve with an increase from 60°C to 95°C at 1°C/3 s. Data were analyzed by using the Fluidigm Real-Time PCR Analysis analysis software and applying the Linear (Derivative) Baseline correction method and the Auto (Global) Ct Threshold method. The Ct values obtained were exported into Excel for the subsequent steps.

The relative assessment of changes in expression was carried out by 2^{-ΔΔCT} method (Livak & Schmittgen, 2001), normalizing the levels of expression of each target gene with respect to the reference gene. Among genes analyzed as possible reference gene (ACTB, B2M, GAPDH, HPRT1, PGK1 and RPLP0) B2M (beta 2-microglobulin) was selected, as constitutively expressed among the samples. Reference gene stability was assessed by programs such as GeNorm (Vandesompele, et al., 2002) and NormFinder (Andersen, et al., 2004). Using alternatively the mean of more reference genes, no relevant variation was observed in the end result. For each individual gene, the increase or the decrease of expression for each patient (fold change), was determined with regard to the mean of the ΔCt obtained from all samples under analysis. The statistical Student's T test was applied on all analyses carried out, and a value of p < 0.05 was considered as significance threshold. The genes that exhibited an at least 2-fold expression difference in the two groups of samples and a p < 0.05 significance value were considered differently expressed. Statistical analysis highlighted ANXA2, STAT1 and NDRG1 as significantly associated with the response to radiochemotherapy treatment, with statistically significant differences in basal levels of tumor expression between sensitive patients and resistant ones.

### Digital PCR

Moreover, the results obtained from analysis by nanofluidic-based qPCR were validated by an absolute quantitation of the three genes found differently expressed in the two groups of patients. To this end, Digital PCR (ThermoFisher Scientific) was used on a selection of samples (10S and 10R). The QuantStudio 3D Digital PCR System (ThermoFisher Scientific) envisages the use of nanofluidic chips, QuantStudio 3D Digital PCR 20K Chip Kit v2, inside which the PCR reaction takes place. In detail, starting from the cDNA obtained as described above, nanofluidic chips were constructed containing the PCR duplex reaction comprised of cDNA, QuantStudio 3D Digital PCR Master Mix v2 and of the probes Taqman VIC and FAM corresponding to the genes (as in Table 3). qPCR was carried out according to the following protocol: 96°C, 10 min; 40 PCR cycles of 60°C, 2 min; 98°C, 30 s; 60°C, 2 min. Thereafter, the number of copies of the transcript was quantitated (as copies/µl of cDNA) through QuantStudio 3D tool, and subsequently the chip was analyzed by software QuantStudio 3D Analysis Suite Cloud. The results obtained were statistically analyzed using the statistical Student's T test, and a value of p < 0.05 was considered as significance threshold.

### RT-qPCR

The expression of the three genes identified as significantly associated to the response to radiochemotherapy treatment was also assessed by RT-qPCR, representing the technology of reference for large-scale test development. The analysis was conducted on a selection of patients (16S and 13R), due to the scarce quantity of RNA for some samples. In detail, 400 ng of RNA were retrotranscribed into cDNA using the iScript cDNA Synthesis Kit (Bio-Rad Laboratories), according to the protocol of the kit. qPCR reaction occurred by using iQ SYBR Green Supermix (Bio-Rad Laboratories) with the Real Time PCR CFX Connect (Bio-Rad Laboratories) detection system. The primers used are the same of the nanofluidic-based qPCR (as reported in Table 2). qPCR was carried out by using the following protocol: 95°C, 3 min; 40 PCR cycles of 95°C, 15 s; 60°C, 45 s and a melting curve with an increase of from 65°C to 95°C at 0.5°C/5 s. All samples were amplified in duplicate and normalized with respect to the reference gene B2M. The relative amounts of mRNA (fold change, FC) were calculated by using the comparative quantitative method of Biorad software based on the 2^{-ΔΔCT} method (Livak and Schmittgen, 2001). For the subsequent bioinformatics analysis, for each sample the ΔCt related to each gene of interest were considered (see below).

### Bioinformatics analysis software

The data set collected on genes ANXA2, NDRG1 and STAT1, through the qPCR technique, was used as "training" dataset (dataset comprised of 29 patients: 13 Resistant "R" and 16 Sensitive "S") to implement and optimize the "Random Forest" (RF) method (Breiman, 2001) useful to the classification of the two groups of patients depending on the response to the therapy. In particular, the RF method is mainly used as a predictive algorithm and has obtained vast popularity and use thanks to its versatilities in the classification and implementation of flexible regressive models. Besides RF, other methods (e.g., the "decision trees")were parallelly tested on this dataset, but the RF method was selected due to its best performance on the training dataset comprised of 29 patients (in particular, an "Area under ROC Curve" of about 0.8) and because of the intrinsic properties of this algorithm, among which the ability to use also incomplete/missing data and autonomously perform the mean of the results of numerous decision trees (in our case, 200). The software environments in which the RF algorithm was implemented, trained and subsequently tested are R ("The R Project for Statistical Computing", https://www.r-project.org/) and Orange (http://orange.biolab.si/), both open source free software for the statistical analysis of data, each other yielding similar results. The RF algorithm "trained" on the 29 initial patients is therefore able to predict the response ("R" or "S") of each new patient from the qPCR values of genes ANXA2, NDRG1 and STAT1 obtained on biopsies collected before the therapy. Specifically, the ΔCt related to each gene of interest were used, using B2M as the reference gene. By alternatively using the mean of more reference genes according to GeNorm or NormFinder, the algorithm is likewise able to correctly predict the response to therapy of the "training" dataset.

**Table 1. List of differential protein spots between the two groups, S and R.**

| | **Master No.** | **Appearance** | **T-test** | **Average ratio sensitive/resistant** |
|---|---|---|---|---|
| **1** | 45 | 41 (63) | 0.038 | 1.96 |
| **2** | 54 | 43 (63) | 0.022 | 1.91 |
| **3** | 210 | 57 (63) | 0.033 | 1.53 |
| **4** | 232 | 49 (63) | 0.029 | 3.13 |
| **5** | 329 | 50 (63) | 0.027 | 1.77 |
| **6** | 358 | 60 (63) | 0.050 | 1.30 |
| **7** | 409 | 61 (63) | 0.045 | 1.64 |
| **8** | 414 | 62 (63) | 0.029 | 1.75 |
| **9** | 416 | 49 (63) | 0.034 | 1.88 |
| **10** | 427 | 51 (63) | 0.006 | 1.70 |
| **11** | 434 | 50 (63) | 0.07 | 1.95 |
| **12** | 448 | 58 (63) | 0.010 | 1.64 |
| **13** | 473 | 40 (63) | 0.055 | 1.57 |
| **14** | 476 | 57 (63) | 0.005 | 1.68 |
| **15** | 493 | 54 (63) | 0.041 | 1.47 |
| **16** | 501 | 59 (63) | 0.031 | 1.30 |
| **17** | 576 | 52 (63) | 0.037 | -1.52 |
| **18** | 702 | 57 (63) | 0.027 | -1.93 |
| **19** | 807 | 47 (63) | 0.051 | -1.58 |
| **20** | 982 | 47 (63) | 0.026 | 1.37 |
| **21** | 1063 | 59 (63) | 0.022 | 1.31 |
| **22** | 1075 | 49 (63) | 0.054 | 1.79 |

### Table 2. List of DELTAgene primers (Fluidigm) used in RT-qPCR

**Table 2. List of DELTAgene primers (Fluidigm) used in RT-qPCR**

| **Target gene** | **Forward primer (5'→3')** | **Reverse primer (5'→3')** | **Amplicon length (bp)** |
|---|---|---|---|
| ACTB | CCAACCGCGAGAAGATGAC (SEQ ID NO:1) | TAGCACAGCCTGGATAGCAA (SEQ ID N0:2) | 80 |
| ALDH1A1 | AGCAGAGCAAACTCCTCTCA (SEQ ID NO:3) | TTCACTACTCCAGGAGGAAACC (SEQ ID NO:4) | 78 |
| ANXA2 | CCTGGAAAATGCTTTCCTGAAC (SEQ ID NO:5) | TGGAGTCATACAGCCGATCA (SEQ ID N0:6) | 80 |
| ARHGAP1 | CCTGAAGTGGGATGACCCATA (SEQ ID NO:7) | TCTTCCGCCCATACTTGTCA (SEQ ID NO:8) | 83 |
| B2M | TTAGCTGTGCTCGCGCTAC (SEQ ID NO:9) | CTCTGCTGGATGACGTGAGTAA (SEQ ID NO:10) | 90 |
| BUB3 | CTGCATACGAGCGTTTCCAAA (SEQ ID NO:11) | GGCTTGGGTCCAAATACTCAAC (SEQ ID NO:12) | 86 |
| CALR | GTTCTACGGTGACGAGGAGAAA (SEQ ID NO:13) | AAACTGGCCGACAGAGCATA (SEQ ID NO:14) | 78 |
| CASP14 | CGAGCTAAGCCCAAGGTGTA (SEQ ID NO:15) | TCTCATCTCCACCTACTGTTTCAC (SEQ ID NO:16) | 82 |
| CNDP2 | TGCCGAACATGACTCCTGAA (SEQ ID NO:17) | ATTGGGGCTGCGTAGTTCA (SEQ ID NO:18) | 83 |
| CP | AGAATGGATGCTCAGCTGTCA (SEQ ID NO:19) | GTTACACTCCTGGACCTGGAAA (SEQ ID NO:20) | 79 |
| GAPDH | GAACGGGAAGCTTGTCATCAA (SEQ ID NO:21) | ATCGCCCCACTTGATTTTGG (SEQ ID NO:22) | 79 |
| GSN | AAGACCTGGCAACGGATGAC (SEQ ID NO:23) | TTGAGAATCCTTTCCAACCCAGAC (SEQ ID NO:24) | 77 |
| HNRNPH 1 | TGTACGGCTTAGAGGACTTCC (SEQ ID NO:25) | TCCCATTTGGCACGATTTCC (SEQ ID NO:26) | 86 |
| HNRNPH 2 | CGCTATAGCCGTTTGAGGGAA (SEQ ID NO:27) | GGCAAGTTTGGCTCAATGCA (SEQ ID NO:28) | 84 |
| HPRT1 | GCTTTCCTTGGTCAGGCAGTA (SEQ ID NO:29) | ACTTCGTGGGGTCCTTTTCAC (SEQ ID NO:30) | 76 |
| HSP90AB 1 | TCTCGCATGAAGGAGACACA (SEQ ID NO:31) | CGCACTCGCTCCACAAAA (SEQ ID NO:32) | 92 |
| HYOU1 | TCTTCACTGAGGTGGAGATGAC (SEQ ID NO:33) | GCCAGAGTTGCATTCTTCCA (SEQ ID NO:34) | 76 |
| LMNA | CGGATGCGCTGCAGGAA (SEQ ID NO:35) | CCAGGTTGCTGTTCCTCTCA (SEQ ID NO:36) | 129 |
| NDRG1 | ATGTACCCCTCCATGGATCA (SEQ ID NO:37) | CTCCTGTTCCCATGCCAATA (SEQ ID NO:38) | 91 |
| PCK2 | GCAAGAAGTGCTTTGCCCTA (SEQ ID NO:39) | AAGGCGGCTGCCACATA (SEQ ID NO:40) | 127 |
| PDCD4 | ACCTGAATTAGCACTGGATACTCC (SEQ ID NO:41) | CCATCTCCAACAGCTCTAGCAA (SEQ ID NO:42) | 75 |
| PGK1 | GTGGAATGGCTTTTACCTTCC (SEQ ID NO:43) | CTTGGCTCCCTCTTCATCAA (SEQ ID NO:44) | 80 |
| RPLP0 | GCGACCTGGAAGTCCAAC (SEQ ID NO:45) | CACATTGTCTGCTCCCACAA (SEQ ID NO:46) | 87 |
| SELENBP1 | GGCAGCTCCCAATGTCTTAC (SEQ ID NO:47) | ACATATAAGTGGCTCCCGTACA (SEQ ID NO:48) | 78 |
| SFN | CTGGACAGCCACCTCATCAA (SEQ ID NO:49) | TAGCGGTAGTAGTCACCCTTCA (SEQ ID NO:50) | 83 |
| STAT1 | ATGCTGGCACCAGAACGAA (SEQ ID NO:51) | GCTGGCACAATTGGGTTTCAA (SEQ ID NO:52) | 84 |
| SYNCRIP | AGCAGCTCAGGAGGCTGTTA (SEQ ID NO:53) | GCAACTGAGATGCAGACACCAA (SEQ ID NO:54) | 81 |
| TGM2 | GCAATGAGTTTGGGGAGATCC (SEQ ID NO:55) | CTGGTCATCCACGACTCCA (SEQ ID NO:56) | 82 |
| TINAGL1 | CATGGGACCCACTCAGTCA (SEQ ID NO:57) | CCAGTATTTGAGCGTCCTTCC (SEQ ID NO:58) | 75 |
| VIM | CCTGTGAAGTGGATGCCCTTA (SEQ ID NO:59) | CAACGGCAAAGTTCTCTTCCA (SEQ ID N060) | 81 |
| XRCCS | GCGTGGCTTTTCCTCATATCA(SEQ ID NO:61) | GTATTGCCGCAAGTCTTCCA (SEQ ID NO:62) | 83 |

**Table 3. List of Taqman probes (ThermoFisher Scientific) used in dPCR.**

| **Target gene** | **Assay ID (ThermoFisher Scientific)** | **Dye** | **Amplicon length (bp)** |
|---|---|---|---|
| ACTB | Hs01060665_g1 | VIC-MGB | 63 |
| ANXA2 | Hs00743063_s1 | FAM-MGB | 68 |
| NDRG1 | Hs00608387_m1 | VIC-MGB | 54 |
| STAT1 | Hs01013996_m1 | FAM-MGB | 66 |

### Action mechanisms

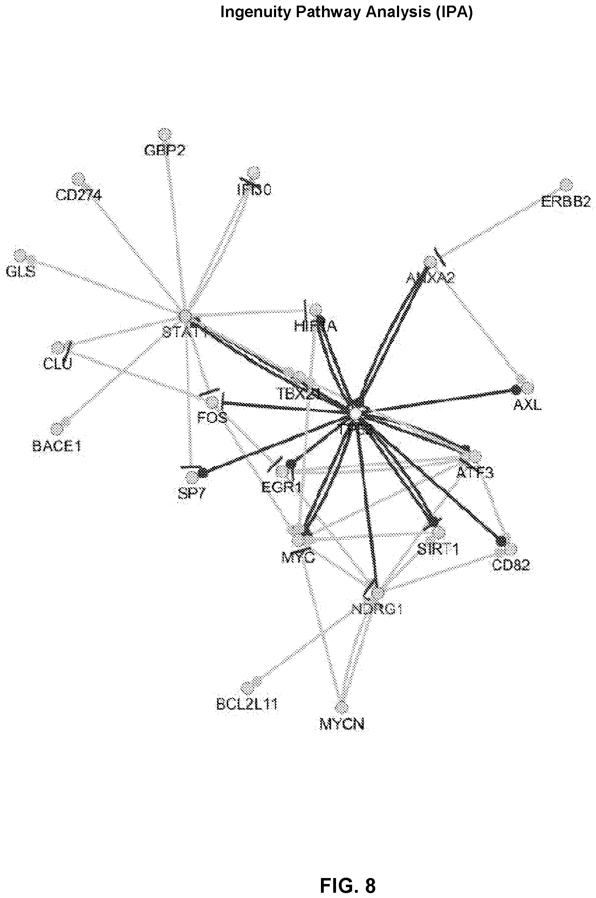

Though not wishing to bind the invention to any theory, on the basis of the experiments the following considerations can be made:
Invasive cervical cancer comprises two main histotypes: squamous cell carcinoma (representing about 80-85% of the cases) and adenocarcinoma (∼10%). Virtually all squamous tumors and most of the adenocarcinomas are HPV-positive. HPV integration into the genome causes an inactivation of the pathways linked to p53 and Rb (among the most important tumor suppressors). In particular as to p53, it is known that the interaction of the protein E6 (of viral origin) with p53 interferes both with the p53 transcription and translation signals and with those leading to apoptosis (Tjalma et al., 2005). In fact, p53 binding by E6 causes inactivation of the former through induction of ubiquitination and therefore degradation by proteasome. Under physiological conditions, p53 is activated following DNA damage and promotes the transcription of p21, a cyclin-dependent inhibitor of the cyclin-kinase complex, which blocks cell cycle progression; alternatively, induces cell apoptosis when DNA damage is irreparable (Vogelstein et al., 2000). Ionizing radiations and cytotoxic drugs, by inducing DNA damage, activate p53 pathway. Pathway inactivation in HPV-positive patients is a key factor in the viral carcinogenesis process but plays a fundamental role also in the response to radiochemotherapy treatment. It is important to stress how all three of the selected proteins converge on the p53 pathway (Fig. 8). STAT1 can induce different types of cell death, and with different mechanisms of action. The leading mechanism of action is the transcriptional regulation of pro-apoptotic genes. However, also non-trascriptional signaling pathways have been demonstrated, among which the interaction with p53 or other proapoptotic factors (Kim and Lee, 2007). NDRG1 represents a direct transcriptional target of p53 and constitutes a protein required, yet not sufficient, in p53-mediated apoptosis (Stein et al., 2004). Other literature data report a NDRG1 role in mediating the resistance to cytotoxic drug-induced apoptosis (Shah et al., 2005; Weiler et al., 2014). However, its biological function is still subject of study. Finally, experimental evidences support a role of ANXA2 as negative regulator of p53 expression (Wang et al., 2012). ANXA2 is moreover a "radioresponsive" protein which prevents radiation-induced apoptosis, by regulating the nuclear translocation of NF-*κ*B (nuclear factor kappa-light-chain-enhancer of activated B cells) (Waters et al., 2013).

### REFERENCES

1. Andersen CL, Jensen JL, Orntoft TF. Normalization of real-time quantitative reverse transcription- PCR data: a model-based variance estimation approach to identify genes suited for normalization, applied to bladder and colon cancer data sets. Cancer Res 2004;64:5245-50.
2. Breiman, L. Random forests. Mach. Learn. 45, 5-32 (2001). DOI 10.1023/A:1010933404324.
3. Livak KJ, Schmittgen TD. Analysis of relative gene expression data using real-time quantitative PCR and the 2(-Delta C(T)) Method. Methods 2001;25:402-8.
4. Vandesompele J, De Preter K, Pattyn F, Poppe B, Van Roy N, De Paepe A, Speleman F. Accurate normalization of real-time quantitative RTPCR data by geometric averaging of multiple internal control genes. Genome Biol 2002;3: RESEARCH0034.

## Claims

1. A method to predict the responsiveness or non-responsiveness to a radiochemotherapy treatment in a patient with a LACC tumor, comprising:
a) determining, in a sample obtained from said patient, the level of expression of the following markers: ANXA2, STAT1 and NDRG1;
b) comparing the level of expression determined in step a) with one or more reference values.

2. The method according to claim 1, wherein the level of expression of said markers ANXA2, STAT1 and NDRG1 is determined by measuring the mRNA or the corresponding protein of said markers in said sample.

3. The method according to any one of claims 1 to 2, wherein the mRNA expression level of said markers is measured by using primers or probes that specifically bind to the sequences of said markers and/or wherein the level of protein expression is measured by using an antibody specific to the corresponding protein of said markers.

4. The method according to any one of claims 1 to 3, wherein said sample is a biopsy sample, in particular a sample obtained from biopsy during diagnostic surgery.

5. The method according to any one of claims 1 to 4, wherein the level of mRNA expression of said markers is determined by qPCR or RT-qPCR.

6. The method according to any one of claims 1 to 5, wherein the level of expression of said markers is normalized by comparing it with the level of expression of one or more reference genes selected from the group consisting of the reference genes ACTB, B2M, GAPDH, HPRT1, PGK1 and/or RPLP0.

7. The method according to any one of claims 1 to 6, wherein said reference value is obtained by analyzing the levels of expression of said markers in one or more samples obtained from a patient with LACC, whose responsiveness or non-responsiveness to a radiochemotherapy treatment is known.

8. The method according to any one of claims 1 to 7, whereby an expression value of the ANXA2 and NDRG1 markers lower than said reference value and an expression value of STAT1 higher than said reference value indicates that said patient is not responsive to a chemotherapy treatment for LACC, wherein said expression value is expressed as ΔCt.

9. The method according to any one of claims 1 to 8, wherein said radiotherapy comprises a local radiant treatment with a dose ranging from 39.6 to 50.4 Gy,

10. The method according to any one of claims 1 to 9, wherein said chemotherapy comprises a treatment with chemotherapeutic drugs selected from cisplatin, 5fluorouracil and/or capecitabine.

11. The method according to any one of claims 1 to 10, wherein said radiochemotherapy comprises a local radiant treatment with a dose ranging from 39.6 to 50.4 Gy in association with a treatment with chemotherapeutic drugs selected from cisplatin, 5-fluorouracil and/or capecitabine.

12. The method according to any one of claims 1 to 4, wherein the value of the expression determined in step a) is applied to a model of prediction of the responsiveness to a radiochemotherapy treatment and wherein said value is compared with the reference value to determine the patient response to radiochemotherapy treatment, in particular wherein said prediction model is created by applying a Random Forest algorithm, wherein the reference values are determined by analyzing the levels of expression of the ANXA2, STAT1 and NDRG1 markers from multiple patients with LACC, constructing a database of the values measured from the patients with known response to radiochemotherapy treatment.

13. The method according to any one of claims 1 to 12, wherein in said step a) are determined in a biopsy sample from said patient by qPCR, the ΔCt of the ANXA2, STAT1 and NDRG1 markers with respect to the reference gene B2M; and wherein the responsiveness to a radiochemotherapy treatment is determined through the application of the "Random Forest" (RF) algorithm.

14. Use of a kit to predict the responsiveness or non-responsiveness to radiochemotherapy treatment in a patient with LACC, comprising agents for measuring the expression level of mRNA or proteins of the following ANXA2, STAT1 and NDRG1 markers,
wherein said agents for measuring the mRNA expression level are pairs of primers or probes that specifically bind to the genes of said markers.

15. Use of the following ANXA2, STAT1 and NDRG1 markers as expression markers to predict the responsiveness or non-responsiveness to radiochemotherapy treatment in a patient with LACC.

## Patentansprüche

1. Verfahren zum Vorhersagen der Ansprechempfindlichkeit oder Nicht-Ansprechempfindlichkeit auf eine Radiochemotherapiebehandlung bei einem Patienten mit einem LACC-Tumor, umfassend:
a) Bestimmen, in einer Probe, die von dem Patienten erhalten wird, des Expressionsniveaus der folgenden Marker: ANXA2, STAT1 und NDRG1;
b) Vergleichen des Expressionsniveaus, das in Schritt a) bestimmt wird, mit einem oder mehreren Referenzwerten.

2. Verfahren nach Anspruch 1, wobei das Expressionsniveau der Marker ANXA2, STAT1 und NDRG1 durch Messen der mRNA oder des entsprechenden Proteins der Marker in der Probe bestimmt wird.

3. Verfahren nach einem der Ansprüche 1 bis 2, wobei das mRNA-Expressionsniveau der Marker durch Verwenden von Primern oder Sonden gemessen wird, die an die Sequenzen der Marker spezifisch binden, und/oder wobei das Proteinexpressionsniveau durch Verwenden eines Antikörpers gemessen wird, der für das entsprechende Protein der Marker spezifisch ist.

4. Verfahren nach einem der Ansprüche 1 bis 3, wobei die Probe eine Biopsieprobe ist, insbesondere eine Probe, die von einer Biopsie während einer diagnostischen Operation erhalten wird.

5. Verfahren nach einem der Ansprüche 1 bis 4, wobei das mRNA-Expressionsniveau der Marker durch qPCR oder RT-qPCR bestimmt wird.

6. Verfahren nach einem der Ansprüche 1 bis 5, wobei das Expressionsniveau der Marker durch Vergleichen dieses mit dem Expressionsniveau eines oder mehrerer Referenzgene normalisiert wird, die aus der Gruppe ausgewählt sind, bestehend aus den Referenzgenen ACTB, B2M, GAPDH, HPRT1, PGK1 und/oder RPLP0.

7. Verfahren nach einem der Ansprüche 1 bis 6, wobei der Referenzwert durch Analysieren der Expressionsniveaus der Marker in einer oder mehreren Proben ermittelt wird, die von einem Patienten mit LACC erhalten werden, dessen Ansprechempfindlichkeit oder Nicht-Ansprechempfindlichkeit auf eine Radiochemotherapiebehandlung bekannt ist.

8. Verfahren nach einem der Ansprüche 1 bis 7, wodurch ein Expressionswert der Marker ANXA2 und NDRG1, der niedriger als der Referenzwert ist, und ein Expressionswert von STAT1, der höher als der Referenzwert ist, anzeigt, dass der Patient nicht auf eine Chemotherapiebehandlung für LACC anspricht, wobei der Expressionswert als ΔCt exprimiert wird.

9. Verfahren nach einem der Ansprüche 1 bis 8, wobei die Radiotherapie eine lokale Strahlenbehandlung mit einer Dosis in einem Bereich von 39,6 bis 50,4 Gy umfasst,

10. Verfahren nach einem der Ansprüche 1 bis 9, wobei die Chemotherapie eine Behandlung mit Chemotherapeutika umfasst, die aus Cisplatin, 5Fluorouracil und/oder Capecitabin ausgewählt sind.

11. Verfahren nach einem der Ansprüche 1 bis 10, wobei die Radiochemotherapie eine lokale Strahlungsbehandlung mit einer Dosis in dem Bereich von 39,6 bis 50,4 Gy in Verbindung mit einer Behandlung mit Chemotherapeutika umfasst, die aus Cisplatin, 5-Fluorouracil und/oder Capecitabin ausgewählt sind.

12. Verfahren nach einem der Ansprüche 1 bis 4, wobei der Wert der Expression, die in Schritt a) bestimmt wird, auf ein Vorhersagemodell der Ansprechempfindlichkeit auf eine Radiochemotherapiebehandlung angewendet wird und wobei der Wert mit dem Referenzwert verglichen wird, um die Reaktion des Patienten auf die Radiochemotherapiebehandlung zu bestimmen, insbesondere wobei dieses Vorhersagemodell durch Anwenden eines Random-Forest-Algorithmus erstellt wird, wobei die Referenzwerte durch Analysieren der Expressionsniveaus der Marker ANXA2, STAT1 und NDRG1 von mehreren Patienten mit LACC bestimmt werden, wobei eine Datenbank der Werte eingerichtet wird, die von den Patienten mit bekannter Reaktion auf die Radiochemotherapiebehandlung gemessen wird.

13. Verfahren nach einem der Ansprüche 1 bis 12, wobei in Schritt a) in einer Biopsieprobe von dem Patienten durch qPCR die ΔCt der Marker ANXA2, STAT1 und NDRG1 hinsichtlich des Referenzgens B2M bestimmt werden; und wobei
die Ansprechempfindlichkeit auf eine Radiochemotherapiebehandlung durch die Anwendung des "Random Forest"-Algorithmus (RF-Algorithmus) bestimmt wird.

14. Verwendung eines Kits, um die Ansprechempfindlichkeit oder Nicht-Ansprechempfindlichkeit auf eine Radiochemotherapiebehandlung bei einem Patienten mit LACC vorherzusagen, umfassend Mittel zum Messen des Expressionsniveaus von mRNA oder Proteinen der folgenden Marker ANXA2, STAT1 und NDRG1,
wobei die Mittel zum Messen des mRNA-Expressionsniveaus Primer- oder Sondenpaare sind, die an die Gene der Marker spezifisch binden.

15. Verwendung der folgenden Marker ANXA2, STAT1 und NDRG1 als Expressionsmarker, um die Ansprechempfindlichkeit oder Nicht-Ansprechempfindlichkeit auf eine Radiochemotherapiebehandlung bei einem Patienten mit LACC vorherzusagen.

## Revendications

1. Procédé permettant de prédire la réactivité ou la non-réactivité à un traitement radiochimique chez un patient atteint d'une tumeur LACC, comprenant :
a) la détermination, dans un échantillon obtenu dudit patient, du niveau d'expression des marqueurs suivants : ANXA2, STAT1 et NDRG1 ;
b) la comparaison du niveau d'expression déterminé à l'étape a) à une ou plusieurs valeurs de référence.

2. Procédé selon la revendication 1, dans lequel le niveau d'expression desdits marqueurs ANXA2, STAT1 et NDRG1 est déterminé en mesurant l'ARNm ou la protéine correspondante desdits marqueurs dans ledit échantillon.

3. Procédé selon l'une quelconque des revendications 1 à 2, dans lequel le niveau d'expression de l'ARNm desdits marqueurs est mesuré à l'aide des amorces ou des sondes qui se lient spécifiquement aux séquences desdits marqueurs et/ou dans lequel le niveau d'expression de la protéine est mesuré à l'aide d'un anticorps spécifique à la protéine correspondante desdits marqueurs.

4. Procédé selon l'une quelconque des revendications 1 à 3, dans lequel ledit échantillon est un échantillon de biopsie, en particulier un échantillon obtenu à partir d'une biopsie au cours d'une chirurgie diagnostique.

5. Procédé selon l'une quelconque des revendications 1 à 4, dans lequel le niveau d'expression de l'ARNm desdits marqueurs est déterminé par qPCR ou RT-qPCR.

6. Procédé selon l'une quelconque des revendications 1 à 5, dans lequel le niveau d'expression desdits marqueurs est normalisé en le comparant au niveau d'expression d'un ou plusieurs gènes de référence choisis dans le groupe constitué des gènes de référence ACTB, B2M, GAPDH, HPRT1, PGK1 et/ou RPLPO.

7. Procédé selon l'une quelconque des revendications 1 à 6, dans lequel ladite valeur de référence est obtenue en analysant les niveaux d'expression desdits marqueurs dans un ou plusieurs échantillons obtenus à partir d'un patient atteint de LACC, dont la réactivité ou la non-réactivité à un traitement de radiochimiothérapie est connue.

8. Procédé selon l'une quelconque des revendications 1 à 7, dans lequel une valeur d'expression des marqueurs ANXA2 et NDRG1 inférieure à ladite valeur de référence et une valeur d'expression de STAT1 supérieure à ladite valeur de référence indiquent que ledit patient ne répond pas à un traitement de chimiothérapie pour le LACC, dans lequel ladite valeur d'expression est exprimée en tant que ΔCt.

9. Procédé selon l'une quelconque des revendications 1 à 8, dans lequel ladite radiothérapie comprend un traitement radiant local avec une dose allant de 39,6 à 50,4 Gy,

10. Procédé selon l'une quelconque des revendications 1 à 9, dans lequel ladite chimiothérapie comprend un traitement avec des médicaments chimiothérapeutiques choisis parmi le cisplatine, le 5fluorouracile et/ou la capécitabine.

11. Procédé selon l'une quelconque des revendications 1 à 10, dans lequel ladite radiochimiothérapie comprend un traitement radiant local avec une dose allant de 39,6 à 50,4 Gy en association avec un traitement par des médicaments chimiothérapeutiques choisis parmi le cisplatine, le 5-fluorouracile et/ou la capécitabine.

12. Procédé selon l'une quelconque des revendications 1 à 4, dans lequel la valeur de l'expression déterminée à l'étape a) est appliquée à un modèle de prédiction de la réactivité à un traitement de radiochimiothérapie et dans lequel cette valeur est comparée à la valeur de référence pour déterminer la réponse du patient au traitement de radiochimiothérapie, en particulier, dans lequel ledit modèle de prédiction est créé en appliquant un algorithme Random Forest, dans lequel les valeurs de référence sont déterminées en analysant les niveaux d'expression des marqueurs ANXA2, STAT1 et NDRG1 de plusieurs patients atteints de LACC, en construisant une base de données des valeurs mesurées chez les patients dont la réponse au traitement par radiochimiothérapie est connue.

13. Procédé selon l'une quelconque des revendications 1 à 12, dans lequel, dans ladite étape a), sont déterminés, dans un échantillon de biopsie dudit patient, par qPCR, le ΔCt des marqueurs ANXA2, STAT1 et NDRG1 par rapport au gène de référence B2M ; et dans lequel
la réactivité à un traitement de radiochimiothérapie est déterminée par l'application de l'algorithme "Random Forest" (RF).

14. Utilisation d'un kit pour prédire la réactivité ou la non réactivité au traitement par radiochimiothérapie chez un patient atteint de LACC, comprenant des agents pour mesurer le niveau d'expression de l'ARNm ou des protéines des marqueurs ANXA2, STAT1 et NDRG1,
dans laquelle les agents de mesure du niveau d'expression de l'ARNm sont des paires d'amorces ou de sondes qui se lient spécifiquement aux gènes desdits marqueurs.

15. Utilisation des marqueurs ANXA2, STAT1 et NDRG1 suivants comme marqueurs d'expression pour prédire la réactivité ou la non-réactivité au traitement par radiochimiothérapie chez un patient atteint de LACC.
